# EUROPEAN PATENT APPLICATION

(11) **EP 4 825 305 A1**
(43) Date of publication of application: **21.10.2026**
(21) Application number: 24903523.9
(22) Date of filing: 29.11.2024
(51) Int. Cl.: A61B 3/10

(54) **IMAGE PROCESSING DEVICE, OPTICAL TOMOGRAPHIC IMAGE IMAGING DEVICE EQUIPPED THEREWITH, AND COMPUTER PROGRAM**

(30) Priority: 13.12.2023 JP 2023210592
(71) Applicant: Tomey Corporation, Nagoya-shi, Aichi 451-0051 (JP)
(72) Inventor: TOTANI, Kota, Nagoya-shi, Aichi 451-0051 (JP); SUGIYAMA, Satoshi, Nagoya-shi, Aichi 451-0051 (JP)
(74) Representative: Winter, Brandl - Partnerschaft mbB
(86) International application number: PCT/JP2024/042309
(87) International publication number: WO 2025/126865

(57) **Abstract**

An image processing apparatus may include: an acquisition unit configured to acquire a plurality of tomographic images obtained by imaging cross-sections at different positions of a subject eye; a display unit configured to display a tomographic image acquired by the acquisition unit; a specifying unit; and a processing unit. The specifying unit may be configured to be operated by a user and to specify boundary positions between layers in the tomographic image displayed on the display unit. The processing unit may be configured to perform an interpolation process to interpolate and determine, when an unspecified tomographic image in which boundary positions have not been specified is present between two specified tomographic images of cross-sections adjacent thereto among specified tomographic images in which the boundary positions have been specified by the specifying unit, a boundary of the unspecified tomographic image based on boundaries in the two specified tomographic images.

## Description

### Technical Field

The technology disclosed in the present specification relates to an image processing apparatus, an optical coherence tomographic apparatus including the image processing apparatus, and a computer program. Specifically, it relates to a technology for processing tomographic images of a subject eye.

### Background Art

An examiner, such as a physician, may use tomographic images of a subject eye to diagnose the condition of the subject eye. For the diagnosis of the subject eye based on the examiner's observation on the tomographic images of the subject eye, drawing boundaries between layers of the subject eye facilitates recognition of the tissues within the subject eye. For example, Patent Document 1 describes an image processing apparatus that sets boundaries between layers of a subject eye.

### Citation List

### Patent Document

[Patent Document 1] Japanese Patent Application Publication No. 2019-88957

### Summary of Invention

### Technical Problem

The image processing apparatus described in Patent Document 1 sets boundaries between layers of a subject eye in tomographic images; however, generally speaking, it is impossible to accurately identify all boundary positions. For example, the image processing apparatus may not accurately identify an interlayer boundary position at a location where a layer boundary is not clearly captured, such as in an affected area. Therefore, a user of the image processing apparatus needs to manually correct boundary positions for such locations with boundary misalignment. Due to the number of tomographic images captured for a single subject eye being enormous, manually correcting boundary positions in every single tomographic image takes a considerable amount of time.

The present specification discloses an image processing apparatus that accurately sets boundaries between layers in tomographic images of a subject eye, while reducing the burden on a user.

### Solution to Technical Problem

In a first aspect of the technology disclosed herein, an image processing apparatus may comprise: an acquisition unit configured to acquire a plurality of tomographic images obtained by imaging cross-sections at different positions of a subject eye; a display unit configured to display a tomographic image acquired by the acquisition unit; a specifying unit; and a processing unit. The specifying unit may be configured to be operated by a user and to specify boundary positions between layers in the tomographic image displayed on the display unit. The processing unit may be configured to perform an interpolation process to interpolate and determine, when an unspecified tomographic image in which boundary positions have not been specified is present between two specified tomographic images of cross-sections adjacent thereto among specified tomographic images in which the boundary positions have been specified by the specifying unit, a boundary of the unspecified tomographic image based on boundaries in the two specified tomographic images.

With the image processing apparatus described above, the boundary in the unspecified tomographic image that is present between the two specified tomographic images is interpolated in the interpolation process based on the boundaries (i.e., corrected boundaries) in the two specified tomographic images on its both sides. Thus, by a user of the image processing apparatus (simply referred to as "the user" hereinafter) simply instructing correction of the boundary positions in the two tomographic images, the boundary in a tomographic image (unspecified tomographic image) interposed between these two tomographic images (specified tomographic images) is corrected to its substantially accurate positions in the interpolation process without having have to instruct any correction for the unspecified tomographic image. This can reduce the number of tomographic images for which the user instructs correction, thereby reducing the user's workload.

In another aspect of the technology disclosed herein, an image processing apparatus may comprise: an acquisition unit configured to acquire a three-dimensional image generated from a plurality of tomographic images obtained by imaging cross-sections at different positions of a subject eye; a display unit configured to display the three-dimensional image acquired by the acquisition unit; a specifying unit; and a processing unit. The specifying unit may be configured to be operated by a user and to specify boundary positions between layers in the three-dimensional image displayed on the display unit. The processing unit may be configured to perform on the three-dimensional image acquired by the acquisition unit: a specific-range specifying process to specify a specific range for correction of boundary positions; and a spatial interpolation process to spatially interpolate and correct the boundary in the specific range based on boundary positions in a non-specific range which is not specified in the specific-range specifying process. The specifying unit specifies the boundary in the three-dimensional image after the spatial interpolation process has been performed. The processing unit may be further configured to spatially interpolate and determine the boundary in the specific range based on the boundary specified by the specifying unit.

The image processing apparatus described above specifies a specific range in the three-dimensional image of the subject eye, and spatially interpolates the boundary in the specific range based on the boundary in the non-specific range. This corrects misalignment of the boundary in the specific range to or near the accurate positions, thereby reducing the number of locations where the user needs to correct the boundary positions. Further, when boundary positions in the specific range are further specified after the boundary in the specific range has been spatially interpolated based on the boundary in the non-specific range (after the spatial interpolation process), the boundary positions are further spatially interpolated based on the specified positions. Thus, boundary misalignment which was not corrected by the spatial interpolation is corrected, thereby accurately specifying the boundary positions.

In another aspect of the technology disclosed herein, an image processing apparatus may comprise: an acquisition unit configured to acquire a plurality of tomographic images obtained by imaging cross-sections at different positions of a subject eye; a processing unit; a display unit; and a correction instruction unit. The processing unit may be configured to: perform, on each of the plurality of tomographic images acquired by the acquisition unit, a segmentation process to determine a boundary between layers by performing segmentation using machine learning; and perform, on each of tomographic images in which the boundary have been determined, a specific-range specifying process to specify a specific range for correction of the boundary positions. The display unit may be configured to display a tomographic image acquired by the acquisition unit and the specific range specified by the processing unit. The correction instruction unit may be configured to be operated by a user and to instruct correction of the boundary positions within the specific range in the tomographic image displayed on the display unit.

In the image processing apparatus described above, the processing unit specifies a specific range to narrow the range in which the user is to correct the boundary positions. Thus, the user's workload for correction of boundary positions can be reduced.

An optical coherence tomographic apparatus disclosed herein may comprise: an imaging capturing unit configured to capture tomographic images along cross-sections at different positions of a subject eye; and any one of the image processing apparatuses described above, configured to process the tomographic images captured by the imaging capturing unit.

The optical coherence tomographic apparatus described above comprises any one of the image processing apparatuses described above. Therefore, the optical coherence tomographic apparatus can achieve the same operational advantages as those of the image processing apparatus described above.

Further, the present specification also discloses a computer program for processing a plurality of tomographic images obtained by imaging cross-sections at different positions of a subject eye. The computer program may cause a computer to function as: an acquisition unit configured to acquire the plurality of tomographic images; a receiving unit configured to receive from a user a specifying instruction to specify boundary positions between layers in each of the tomographic images; and an interpolation unit configured to interpolate and determine, when an unspecified tomographic image in which boundary positions have not been specified is present between two specified tomographic images of cross-sections adjacent thereto among specified tomographic images in which the boundary positions have been specified according to the specifying instruction received from the receiving unit, a boundary of the unspecified tomographic image based on boundaries in the two specified tomographic images.

### Brief Description of Drawings

[FIG. 1] A block diagram illustrating a schematic configuration of an optical coherence tomographic apparatus according to first to fourth embodiments.
[FIG. 2] A flowchart illustrating an example of a process for an image processing apparatus to correct boundaries between layers in a tomographic image of a subject eye, according to the first embodiment.
[FIG. 3] A diagram illustrating an en-face image with a mark indicating a specific range superimposed thereon.
[FIG. 4] Diagrams illustrating tomographic images with a boundary superimposed thereon, where (a) is a tomographic image with a boundary specified in a segmentation process superimposed thereon, and (b) is a tomographic image after boundary positions in the tomographic image (a) have been corrected.
[FIG. 5] A flowchart illustrating an example of a process for an image processing apparatus to correct boundaries between layers in a tomographic image of a subject eye, according to the fourth embodiment.
[FIG. 6] An en-face image showing a specific range and a non-specific range, where weighing in a first spatial interpolation process is indicated by thicknesses of arrows.
[FIG. 7] En-face images each showing a specific range and a non-specific range, where (a) adds a correct position to FIG. 6, and (b) shows weighing in a second spatial interpolation process by thicknesses of arrows.
[FIG. 8] A diagram illustrating an example of an image displayed on a display unit for input of a correct position for a boundary according to a fifth embodiment.
[FIG. 9] An enlarged view of a principal area IX in FIG. 8.
[FIG. 10] An enlarged view of a principal area X in FIG. 8.
[FIG. 11] A diagram illustrating an example of an image in which a specific range is specified using a polarization tomographic image, according to a fifth embodiment.
[FIG. 12] A diagram illustrating an example of an image after a boundary position in a specified range has been corrected.

Some of the features characteristic to below-described embodiments are herein listed. It should be noted that the respective technical elements are independent of one another, and are useful solely or in combinations. The combinations thereof are not limited to those described in the claims as originally filed.

In a second aspect of the technology disclosed in the present specification according to the first aspect, the processing unit may be further configured to perform a segmentation process to determine a boundary between layers by performing segmentation using machine learning on each of the plurality of tomographic images acquired by the acquisition unit. The display unit may display a tomographic image on which the boundary determined in the segmentation process is superimposed. The specifying unit may instruct correction of the boundary positions superimposed on the tomographic image displayed on the display unit. According to this configuration, the processing unit automatically determines the boundary using machine learning. Thus, it is possible to obtain multiple tomographic images in which boundary positions are specified with relatively high accuracy, and the user's workload for correction of boundary positions can be reduced.

In a third aspect of the technology disclosed in the present specification according to the second aspect, the plurality of tomographic images may comprise a non-polarization tomographic image indicating tissues in the subject eye by scattering intensity and a polarization tomographic image indicating a polarization state in the subject eye. The processing unit may perform the segmentation process based on the non-polarization tomographic image and the polarization tomographic image. According to this configuration, an increased amount of information that can be referred to for boundary positions is provided. Thus, the processing unit can determine boundary positions with higher accuracy in the segmentation process.

In a fourth aspect of the technology disclosed in the present specification according to the second or third aspect, the processing unit may be further configured to perform, on each of the tomographic images in which the boundary has been determined in the segmentation process, a specific-range specifying process to specify a specific range for correction of the boundary positions. According to this configuration, by the processing unit specifying a specific range, the range in which the user is to correct the boundary positions can be narrowed to the specific range. Thus, the user's workload can be reduced.

In a fifth aspect of the technology disclosed in the present specification according to the fourth aspect, the processing unit may be further configured to calculate reliability information related to reliability of the boundary positions determined in the segmentation process. In the specific-range specifying process, the processing unit may specify the specific range based on the calculated reliability information such that low-reliability boundary positions are included therein. In locations where the reliability of boundary positions determined in the segmentation process is low, the boundary is highly likely misaligned. By specifying the specific range such that it includes such low-reliability locations, the range in which the user is to correct the boundary positions can be narrowed to the range where boundaries are highly likely misaligned.

In a sixth aspect of the technology disclosed in the present specification according to the fourth aspect, when positions of two adjacent pixels on a boundary determined in the segmentation process differ in a depth direction by a predetermined value or more, the processing unit may specify the specific range in the specific-range specifying process such that the two adjacent pixels are included therein. A boundary gently curves in many portions, and when positions of adjacent pixels on the boundary differ significantly, that is highly likely to indicate a site with a characteristic morphology or an affected site. Since it is difficult to automatically draw a boundary over such a site with a characteristic morphology or an affected site, a boundary is highly likely misaligned there. By specifying the specific range such that it includes adjacent pixels on a boundary that significantly differ in position, the range in which the user is to correct the boundary positions can be narrowed to the range where boundaries are highly likely misaligned.

In a seventh aspect of the technology disclosed in the present specification according to the fourth aspect, in the specific-range specifying process, the processing unit may specify the specific range such that pixels having a luminance value equal to or less than a predetermined value on a boundary determined in the segmentation process are included therein. When a boundary determined in the segmentation process is misaligned from a layer boundary within the subject eye, that boundary have a low luminance value. By specifying the specific range such that it includes pixels having a luminance value equal to or less than the predetermined value, the range in which the user is to correct the boundary positions can be narrowed to the range where boundary is highly likely misaligned.

In an eighth aspect of the technology disclosed in the present specification according to the fourth aspect, the processing unit may be further configured to perform a poor-fixation detection process to detect a location of poor fixation in each of the plurality of tomographic images. The processing unit may specify the specific range such that the location of poor fixation detected in the poor-fixation detection process is included therein. At the location of poor fixation, the subject eye is prone to shifting in the depth direction, and thus the boundary is highly likely misaligned. By specifying the specific range such that it includes the locations of poor fixation, the range in which the user is to correct the boundary positions can be narrowed to the range where boundary is highly likely misaligned.

In a ninth aspect of the technology disclosed in the present specification according to any one of the fourth to eighth aspects, the plurality of tomographic images may comprise a non-polarization tomographic image indicating tissues in the subject eye by scattering intensity and a polarization tomographic image indicating a polarization state in the subject eye. The processing unit may perform the specific-range specifying process based on the non-polarization tomographic image and the polarization tomographic image. According to this configuration, an increased amount of information that can be referred to for boundary positions is provided. Thus, the processing unit can specify the specific range with higher accuracy in the specific-range specifying process.

In a tenth aspect of the technology disclosed in the present specification according to any one of the second to ninth aspects, the processing unit may be configured to perform a specific tomographic image specifying process to specify a specific tomographic image in which boundary positions are to be corrected from among the plurality of tomographic images. According to this configuration, by the processing unit specifying the specific tomographic image, the user simply corrects boundary positions only in the specified tomographic image (i.e., the specific tomographic image) among the plurality of tomographic images. Thus, the user's workload can be reduced.

In an eleventh aspect of the technology disclosed in the present specification according to the tenth aspect, the processing unit may be further configured to calculate reliability information related to reliability of the boundary positions determined in the segmentation process. In the specific tomographic image specifying process, the processing unit may specify, based on the calculated reliability information, a tomographic image including low-reliability boundary positions as the specific tomographic image.

In a twelfth aspect of the technology disclosed in the present specification according to the tenth aspect, in the specific tomographic image specifying process, the processing unit may specify a tomographic image in which positions of two adjacent pixels on a boundary determined in the segmentation process differ in a depth direction by a predetermined value or more as the specific tomographic image.

In a thirteenth aspect of the technology disclosed in the present specification according to the tenth aspect, in the specific tomographic image specifying process, the processing unit may specify a tomographic image that includes pixels having a luminance value equal to or less than a predetermined value on a boundary determined in the segmentation process as the specific tomographic image.

In a fourteenth aspect of the technology disclosed in the present specification according to the tenth aspect, the processing unit may be further configured to perform a poor-fixation detection process to detect a location of poor fixation in each of the plurality of tomographic images. The processing unit may specify a tomographic image including the poor fixation detected in the poor-fixation detection process as the specific tomographic image.

In a fifteenth aspect of the technology disclosed in the present specification according to any one of the tenth to fourteenth aspects, the plurality of tomographic images may comprise a non-polarization tomographic image indicating tissues in the subject eye by scattering intensity and a polarization tomographic image indicating a polarization state in the subject eye. The processing unit may perform the specific tomographic image specifying process based on the non-polarization tomographic image and the polarization tomographic image. According to this configuration, an increased amount of information that can be referred to for boundary positions is provided. Thus, the processing unit can specify the specific tomographic image with higher accuracy in the specific tomographic image specifying process.

In a sixteenth aspect of the technology disclosed in the present specification according to the first or second aspect, the specifying unit may specify, in accordance with user operation, a specific range within the subject eye in the tomographic image and instructs correction of boundary positions within the specified specific range. According to this configuration, a range determined by the user as including misaligned boundary is specified by the user as the specific range. Thus, the range in which the user is to correct the boundary positions can be narrowed to the specific range, thereby reducing the user's workload.

In a seventeenth aspect of the technology disclosed in the present specification according to any one of the first to sixteenth aspects, the plurality of tomographic images may comprise a non-polarization tomographic image indicating tissues in the subject eye by scattering intensity and a polarization tomographic image indicating a polarization state in the subject eye. The display unit may display the non-polarization tomographic image and the polarization tomographic image. According to this configuration, an increased amount of information that can be referred to for boundary positions is displayed on the display unit. Thus, the user can correct the boundary positions with higher accuracy.

### Embodiments

### (First Embodiment)

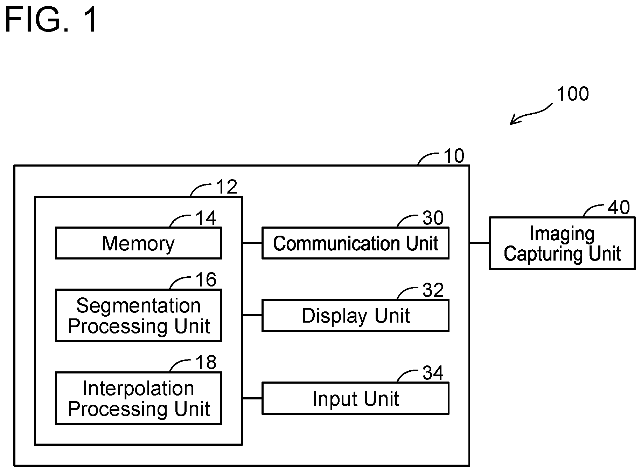

Referring to the drawings, an optical coherence tomographic apparatus 100 according to an embodiment is described. As illustrated in FIG. 1, the optical coherence tomographic apparatus 100 comprises an image processing apparatus 10 and an image capturing unit 40. The image capturing unit 40 captures tomographic images of a subject eye using optical coherence tomography (OCT). The image capturing unit 40 only needs to be capable of capturing tomographic images of a subject eye, and is not limited to units that use optical coherence tomography; further, in case of using OCT, the type of OCT is not particularly limited. For example, when the image capturing unit 40 uses optical coherence tomography, the image capturing unit 40 may be configured to capture tomographic images indicating tissues in the subject eye by scattering intensity (so-called normal tomographic images) or to capture tomographic images indicating a polarization state within the subject eye (hereinafter referred to as polarization tomographic images). When capturing the polarization tomographic images, the image capturing unit 40 may simultaneously obtain tomographic images captured by irradiating the subject eye with vertically polarized light and tomographic images captured by irradiating the subject eye with horizontally polarized light. By using these two types of tomographic images, not only the normal tomographic images but also the polarization tomographic images can be generated. The polarization tomographic images include, for example, tomographic images indicating entropy within the subject eye, tomographic images indicating birefringence within the subject eye, and the like. Detailed description of the image capturing unit 40 is omitted herein because image capturing units used in generally known optical coherence tomographic apparatuses can be used as the image capturing unit 40.

The image processing apparatus 10 comprises a processing unit 12, a communication unit 30, a display unit 32, and an input unit 34. The processing unit 12 is connected to each of the communication unit 30, the display unit 32, and the input unit 34 via wires such as busbars so as to be communicable bidirectionally.

The processing unit 12 comprises a memory 14 and a computer including a CPU (not illustrated). The memory 14 comprises hardware such as a ROM, a RAM, and the like. A processing program is stored in the memory 14, and by the CPU executing the processing program, the processing unit 12 is caused to function as a segmentation processing unit 16, an interpolation processing unit 18, and the like illustrated in FIG. 1. Processes performed by the segmentation processing unit 16 and the interpolation processing unit 18 will be described later. Further, the memory 14 also stores features for identifying layer boundaries in tomographic images of eyes, and these features have been obtained by machine learning performed in advance. These features are used for the segmentation processing unit 16 to perform a segmentation process based on machine learning, which will be described later.

The communication unit 30 comprises a communication interface for wired or wireless communication between the image processing apparatus 10 and an external device. The processing unit 12 can acquire various data from external sources outside the image processing apparatus 10 via the communication unit 30. Specifically, the communication unit 30 acquires tomographic images of a subject eye captured by the image capturing unit 40 from the image capturing unit 40. The communication unit 30 may be connected to an external device outside the optical coherence tomographic apparatus 100 to acquire tomographic images of a subject eye and/or other data from the external device.

In the present embodiment, the display unit 32 is a monitor and displays a tomographic image of the subject eye (specifically, a normal tomographic image and a polarization tomographic image), an en-face image generated from the tomographic images of the subject eye, and a three-dimensional image. The display unit 32 also displays the tomographic image of the subject eye and the three-dimensional image with layer boundaries superimposed thereon.

In the present embodiment, the input unit 34 is a mouse and is configured to input a correct position for a boundary in a tomographic image of the subject eye displayed on the display unit 32. In the present application, a user of the image processing apparatus 10 (hereinafter simply referred to as "the user") can see the tomographic image of the subject eye displayed on the display unit 32 and determine whether the position of boundary superimposed thereon is misaligned or not. When determining that the boundary position is misaligned, the user can input the correct position for the boundary using the input unit 34 so that the boundary is shifted to a layer boundary position (i.e., to the non-misaligned, correct position).

Now, a process performed by the image processing apparatus 10 to correct layer boundaries in a tomographic image of the subject eye is described. As will be described later, in the present embodiment, the processing unit 12 automatically specifies layer boundaries in each of the tomographic images of the subject eye based on machine learning. The automatically specified layer boundaries are generally at their correct positions, but they may be partially misaligned. For example, the processing unit 12 can accurately specify positions of layer boundaries in locations where the layers in the subject eye are clearly captured. However, if the subject eye has an affected site, layer boundaries in the affected site may not be clearly captured. The processing unit 12 cannot accurately specify boundary positions in locations where layer boundaries are not clearly captured, such as in affected sites, which may result in misalignment of boundary positions or discontinuous boundaries due to the failure of boundary specification. Layer boundaries in the subject eye are gently curved in many portions thereof; however, in sites having characteristic morphologies, such as macula and optic disc, the positions of layer boundaries significantly shift. For such sites, the processing unit 12 has difficulty in accurately specifying boundaries, and boundary positions may be misaligned. When the automatically specified boundaries are misaligned, the user needs to manually correct the boundary positions based on the tomographic images of the subject eye. The greater the number of locations that require correction by the user, the greater the user's workload. Hereinafter, a process for correcting positions of layer boundaries in tomographic images of the subject eye, which reduces the number of locations where layer boundaries require correction by the user, is described.

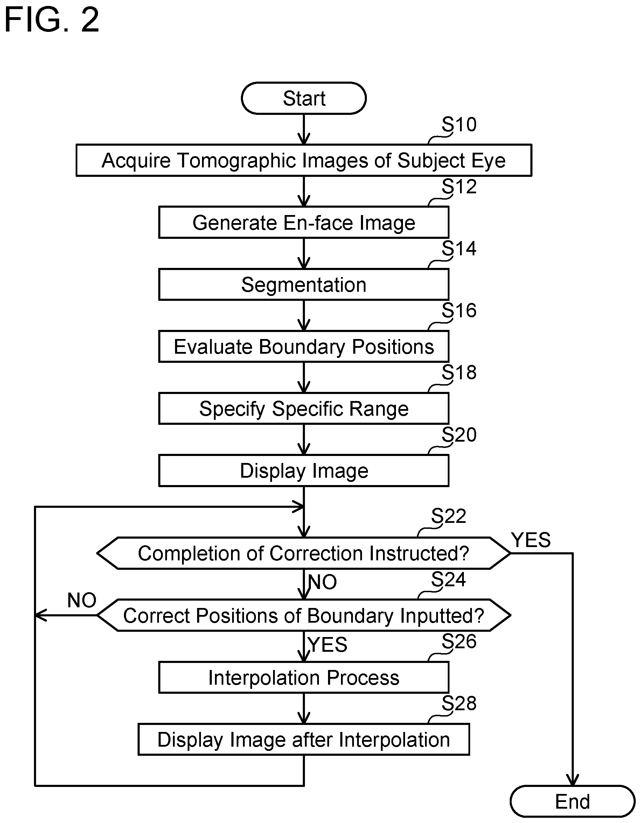

As illustrated in FIG. 2, the processing unit 12 first acquires tomographic images of a predetermined region of the subject eye (in the present embodiment, the retina thereof) (S10). In the present embodiment, the image capturing unit 40 captures 256 tomographic images for 256 directions into which the subject eye is divided (sectioned), and the processing unit 12 acquires the 256 tomographic images from the image capturing unit 40 via the communication unit 30. The number of tomographic images of the subject eye (i.e., the number of divisions of the subject eye and image captures) is not particularly limited.

Subsequently, the processing unit 12 generates an en-face image of the subject eye from the plurality of tomographic images acquired in step S10 (S12). Specifically, the processing unit 12 generates the en-face image by compressing a three-dimensional image, which is generated from the plurality of tomographic images of the subject eye, into a two-dimensional front-view image. In step S10, the processing unit 12 may acquire tomographic images of the subject eye from an external device outside the optical coherence tomographic apparatus 100 via the communication unit 30. If acquiring tomographic images of the subject eye from an external device outside the optical coherence tomographic apparatus 100 in step S10, the processing unit 12 may acquire not only the tomographic images of the subject eye but also an en-face image of the subject eye generated from these tomographic images.

Subsequently, the segmentation processing unit 16 performs a segmentation process on each of the plurality of tomographic images, using machine learning (S14). Specifically, the segmentation processing unit 16 uses the features for determining layer boundaries, which are stored in the memory 14, to specify layer boundaries in each of the tomographic images acquired in step S10. Detailed description of the machine learning used in the segmentation process is omitted herein because generally known machine learning techniques can be used. By performing the segmentation process using machine learning, the processing unit 12 can specify positions of layer boundaries with high accuracy. In step S14, the processing unit 12 may perform the segmentation process using a known method (e.g., the known method disclosed in Japanese Patent Application Publication No. 2019-88957) rather than machine learning, instead of performing the segmentation process using machine learning.

Subsequently, the processing unit 12 evaluates accuracy of the boundary positions specified in step S14 (S16). As described above, while the segmentation process using machine learning allows positions of layer boundaries to be specified with relatively high accuracy, it is still difficult to accurately specify all boundary positions in the plurality of tomographic images. The processing unit 12 evaluates accuracy of the boundary positions specified in step S14 to estimate misalignment of the boundary positions specified in step S14 (specifically, locations where boundaries are highly likely misaligned).

Specifically, the processing unit 12 calculates reliability in specifying the boundary positions using machine learning in step S14. For example, the processing unit 12 can accurately specify, based on machine learning, boundaries in locations where layers are clearly captured without any characteristic morphologies. Accordingly, the reliability is high. In contrast, the processing unit 12 has difficulty in accurately specifying boundaries in locations where layers are not clearly captured and locations having characteristic morphologies. Accordingly, the reliability is low.

Subsequently, the processing unit 12 specifies, based on the evaluation result in step S16, a range including a low-evaluation location (hereinafter referred to as the specific range) (S18). Specifically, the processing unit 12 specifies the specific range such that the low-evaluation location (also referred to as low-reliability location in the present embodiment) and surrounding locations are included in the specific range.

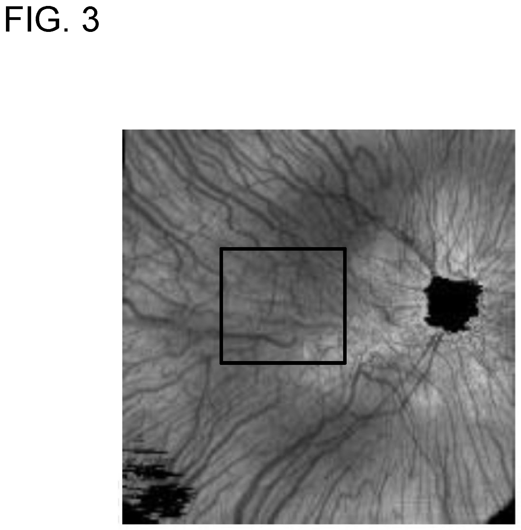

Subsequently, the processing unit 12 causes the display unit 32 to display the en-face image showing the specific range specified in step S18 and a tomographic image of the subject eye including the specific range (S20). For example, as illustrated in FIG. 3, the processing unit 12 causes the display unit 32 to display the en-face image with a mark indicating a specific range (see the square mark in FIG. 3) superposed thereon. The specific range superimposed on the en-face image may be configured to be changed to another one by the user via the input unit 34. Further, the processing unit 12 can cause the display unit 32 to display a tomographic image including the specific range. The tomographic image displayed on the display unit 32 can be selected and switched by the user via the input unit 34. By displaying the en-face image with the specific range superimposed thereon on the display unit 32, the user can be notified of the range where a boundary is highly likely misaligned. The user can simply check misalignment of boundary position in the narrowed range where a boundary is highly likely misaligned, thereby reducing the user's workload.

Subsequently, the interpolation processing unit 18 determines whether a correction completion instruction has been input or not (S22). The user checks boundary position misalignment within the specific range for each of the plurality of tomographic images. After determining that there is no boundary position misalignment within the specific range, the user instructs correction completion. For example, the user instructs correction completion by clicking a "Complete" button on display unit 32 using the input unit 34. After the correction completion instruction has been input (YES in step S22), the process of correcting layer boundaries in the tomographic images of the subject eye, which is illustrated in FIG. 2, ends.

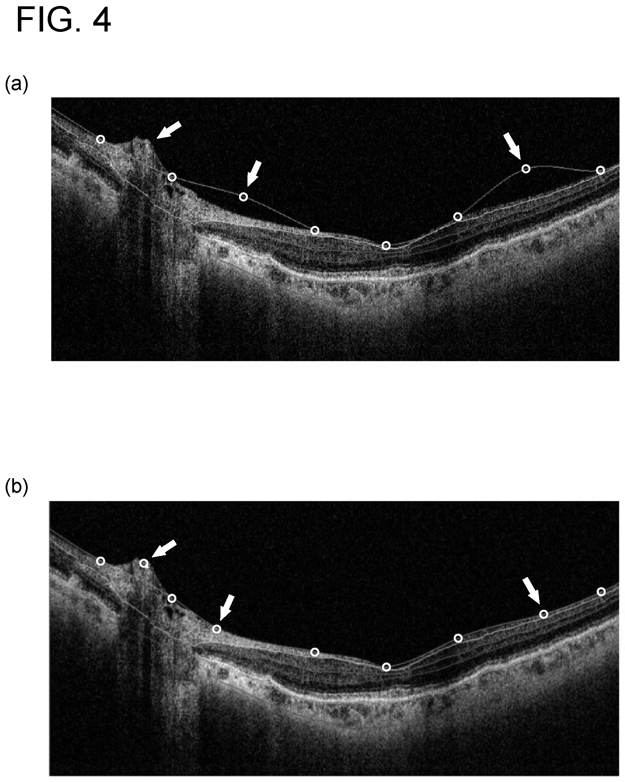

If the correction completion instruction has not been input (NO in step S22), the processing unit 12 determines whether correct positions of boundaries have been input or not (S24). When the user checks boundary position misalignment within the specific range in each of the plurality of tomographic images and determines that there is boundary position misalignment, the user inputs the correct position of the boundary (i.e., corrected position) via the input unit 34. For example, FIG. 4(a) shows a tomographic image with boundaries specified in the segmentation process of step S14 superimposed thereon. In FIG. 4(a), eight marks (circles) are added on the uppermost boundary, for ease of description. As illustrated in FIG. 4(a), the boundary specified in the segmentation process of step S14 includes locations positioned on a layer boundary (the locations indicated by the first, second, fourth, fifth, sixth, and eighth marks from the left in FIG. 4(a)) and locations misaligned from the layer boundary (the locations indicated by the third and seventh marks from the left, and the location between the first and second marks from the left (the locations pointed by the arrows) in FIG. 4(a)). As illustrated in FIG. 4(b), the user input the correct position of the boundary via the input unit 34 such that the boundary's misaligned locations are shifted to the layer boundary.

For the input of correct position of boundary, the user simply inputs the correct positions for boundaries in several tomographic images selected from the plurality of tomographic images. For example, the user checks the 256 tomographic images one by one to select several tomographic images that include positional misalignment or large positional misalignment, and may input the correct positions only for the boundaries in the selected number of tomographic images. Alternatively, the user may input correct positions for boundaries within the specific range in every few tomographic images (e.g., every five tomographic images) out of the 256 tomographic images. Hereinafter, a tomographic image in which the correct position of a boundary has been input may be referred to as a "specified tomographic image", and a tomographic image in which the correct position of a boundary has not been input may be referred to as an "unspecified tomographic image". After completing the input of correct positions of boundaries, the user instructs input completion. For example, before inputting the correct positions of boundaries, the user instructs the start of input of the correct positions of boundaries by clicking a "Correct" button on the display unit 32 via the input unit 34. Then, after completing the input of the correct positions of boundaries, the user instructs the completion of the input of the correct positions of boundaries by clicking the "Correct" button on the display unit 32 again via the input unit 34. If the correct positions of boundaries have not been input (NO in step S24), the flow returns to step S22, and step S22 and step S24 are repeated.

After the input of the correct positions of boundaries (YES in step S24), the interpolation processing unit 18 performs an interpolation process based on the correct positions of boundaries input in step S24 (S28). Specifically, the interpolation processing unit 18 interpolates and corrects, based on the correct positions inputted in a specified tomographic image, boundary positions therearound. Further, the interpolation processing unit 18 also interpolates and corrects boundary positions in an unspecified tomographic image between two specified tomographic images based on the correct positions of boundaries in the two specified tomographic images. Detailed description of the interpolation process is omitted herein because known interpolation processes can be used.

After the completion of the interpolation process, the processing unit 12 displays a tomographic image on which the interpolation process has been performed on the display unit 32 (S28). Subsequently, the flow returns to step S22, and the sequence from step S22 to step S28 is repeated. That is, the user checks the tomographic image on which the interpolation process has been performed, and if the boundary positions are still misaligned, inputs the correct positions of boundary again. The processing unit 12 then interpolates and corrects the boundary position based on the correct position of boundary input again. These steps are repeated until the user determines that no boundary misalignment remains (until YES is determined in step S22).

In the present embodiment, the user inputs the boundary correction only to a limited number of tomographic images (specified tomographic images) selected from the plurality of tomographic images, and the interpolation processing unit 18 interpolates and corrects the boundary positions in an unspecified tomographic image based on the correct positions of boundaries in the specified tomographic images. Thus, the boundary positions in all the plurality of tomographic images can be corrected without the user inputting the correct positions of boundaries to all of the tomographic images. This reduces the user's workload. Further, the processing unit 12 evaluates accuracy of boundary positions specified in the segmentation process of step S14 and specifies a specific range such that low-evaluation locations are included therein. Thus, the user only needs to input the correct positions of the boundaries only for the specific range, thereby reducing the user's workload.

In the present embodiment, in step S16 of evaluating the accuracy of boundary positions specified in the segmentation process of step S14, the accuracy is evaluated by calculating the reliability in specifying the boundary positions using machine learning; however, the evaluation is not limited thereto. Hereinafter, examples of other evaluation methods for accuracy of boundary positions specified in the segmentation process of step S14 are described.

For example, when positions of adjacent pixels on a boundary specified in the segmentation process differ in the depth direction in a tomographic image, the processing unit 12 may evaluate the accuracy of boundary positions for these two adjacent pixels is low. As noted above, the layers in the subject eye gently curve in many portions. However, a boundary position may shift significantly in a site having a characteristic morphology such as macula or optic disc as well as in an affected site. In such a portion of a boundary where adjacent locations are significantly different, it is difficult to accurately specify the boundary positions in the segmentation process. Thus, the boundary positions specified in the segmentation process is highly likely misaligned in such portions. Therefore, when positions of adjacent pixels on a boundary differ in the depth direction by a predetermined value or more, the processing unit 12 may evaluate that the accuracy of the boundary position is low for these two pixels. With this method as well, the user can correct the boundary position in a narrowed range where the boundary position shifts significantly, thereby accurately correcting the boundary position while reducing the user's workload.

The processing unit 12 may evaluate the accuracy of a boundary position based on luminance values on the boundary specified in the segmentation process. Specifically, when a luminance value on a boundary specified in the segmentation process is equal to or less than a predetermined value, the processing unit 12 may evaluate that the accuracy of the boundary position is low. As shown in FIG. 4(a), when a boundary position is misaligned and the boundary extends over areas where no tissue is present, luminance values on the boundary may be low (e.g., see the locations indicated by the middle and right arrows in FIG. 4(a)). Alternatively, if the entire boundary extends within a single tissue, light is not reflected at locations where the tissue changes; therefore, luminance values on such locations of the boundary are low. That is, a boundary is highly likely misaligned in its locations having luminance values less than the predetermined value. Therefore, when luminance values on a boundary are equal to or less than the predetermined value, the accuracy of boundary position may be evaluated as low. With this method as well, the user can correct the boundary position in a narrowed range where the boundary is highly likely misaligned, thereby accurately correcting the boundary position while reducing the user's workload.

The processing unit 12 may specify a location of poor fixation from the plurality of tomographic images and evaluate that the accuracy of boundary position is low for the location of poor fixation. For example, poor fixation is caused by fixation shift or blinking. Locations of poor fixation are likely to be captured with misalignment in the depth direction. Therefore, the accuracy of boundary position may be evaluated as low for such locations of poor fixation. Specifically, the processing unit 12 compares the plurality of tomographic images to specify an image where the position of the subject eye is misaligned in the depth direction. The image with the misalignment in the depth direction is highly likely to have been captured with poor fixation. The processing unit 12 also compares the plurality of tomographic images to specify an image having low overall luminance. The image having low overall luminance is highly likely to have been captured with blinking. The processing unit 12 evaluates that the accuracy of boundary positions is low in the specified images as well as in locations within the specified images misaligned in the depth direction relative to other images. With this method as well, the user can correct the boundary positions in a narrowed range where the boundary is highly likely misaligned, thereby accurately correcting the boundary position while reducing the user's workload.

### (Second Embodiment)

In the first embodiment above, the processing unit 12 specifies a specific range based on the evaluation results of accuracy of boundary positions in step S18; however, the present disclosure is not limited to such a configuration. For example, instead of specifying a specific range based on the evaluation results of accuracy of boundary positions, the processing unit 12 may specify specific tomographic images based on the evaluation results of accuracy of boundary positions. Specifically, the processing unit 12 specifies tomographic images that include many portions with low accuracy evaluation on boundary positions as the specific tomographic images. Then, in step S24, the user inputs correct boundary positions to the specified specific tomographic images. Even in such a case, the user only needs to input correct boundary positions to a limited number of specified tomographic images (i.e., the specific tomographic images) among the plurality of tomographic images, facilitating accurate correction of the boundary positions while reducing the user's workload.

### (Third Embodiment)

In the first embodiment above, the processing unit 12 specifies a specific range in step S18; however, the present disclosure is not limited to such a configuration. For example, instead of the processing unit 12 specifying a specific range, the user may specify a specific range. In this case, in the flowchart of FIG. 2, the processing unit 12 determines whether a specific range has been input or not instead of performing steps S16 and S18, and then performs step S20 onward after a specific range has been input. For example, the user checks the en-face image generated in step S12 to specify a range where a boundary is highly likely misaligned, and inputs that range using the input unit 34. The range where a boundary position is highly likely misaligned often includes, for example, a site having characteristic morphology such as the macula or the optic disc, or a site that is presumed to be affected by a disease. If the user is a medical professional such as a physician, the user can identify, from the en-face image, the range where a boundary position is highly likely misaligned. Even in the case where the user specifies a specific range, the user only needs to input a boundary correct position for the specific range, thereby reducing the user's workload.

### (Fourth Embodiment)

In the first to third embodiments above, boundary positions are corrected by interpolation in the same tomographic image or interpolation between tomographic images; however, the present disclosure is not limited to such a configuration. For example, boundary positions may be corrected by spatial interpolation in a three-dimensional image generated from the plurality of tomographic images. A process of correcting layer boundaries in a three-dimensional image by spatial interpolation will be described below.

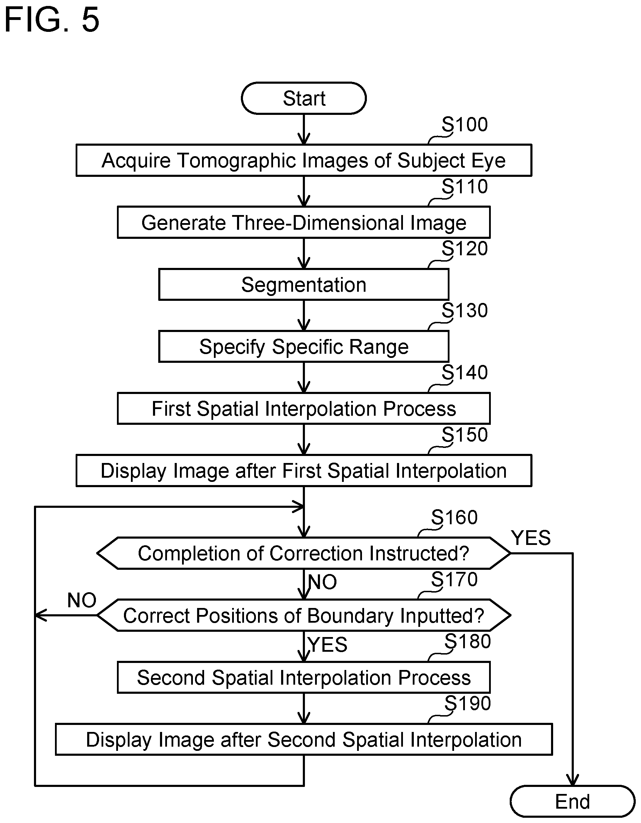

As illustrated in FIG. 5, the processing unit 12 first acquires tomographic images of the subject eye (S100). Since step S100 is the same as step S10 in the first embodiment, detailed description thereof is omitted.

Subsequently, the processing unit 12 generates a three-dimensional image of the subject eye from the plurality of tomographic images acquired in step S100 (S110). The processing unit 12 may acquire tomographic images of the subject eye from a device outside the optical coherence tomographic apparatus 100 via the communication unit 30 in step S100. If the processing unit 12 acquires tomographic images of the subject eye from a device outside the optical coherence tomographic apparatus 100 in step S100, the processing unit 12 may also acquire, together with the tomographic images of the subject eye, a three-dimensional image of the subject eye generated from these tomographic images.

Subsequently, the segmentation processing unit 16 performs the segmentation process on each of the plurality of tomographic images based on machine learning (S120). Since step S120 is the same as step S14 in the first embodiment, detailed description thereof is omitted.

Subsequently, the processing unit 12 specifies a specific range (S130). The specific range is a range that includes portions where boundary positions are highly likely misaligned. The specific range may be specified by the processing unit 12 based on evaluation result of boundary position accuracy, or may be specified by the user inputting the specific range using the input unit 34. Since specifying of the specific range by the processing unit 12 here is the same as steps S16 and S18 in the first embodiment, detailed description thereof is omitted.

Subsequently, the interpolation processing unit 18 performs a spatial interpolation process (hereinafter referred to as a first spatial interpolation process) for boundary positions within the specific range specified in step S130 based on boundary positions within a range outside the specific range specified in step S130 (hereinafter referred to as a non-specific range) (S140). Since the spatial interpolation process here can be performed using publicly known spatial interpolation processing technology, detailed description thereof is omitted. As described above, the specific range includes portions where boundary positions are highly likely misaligned. That is, the non-specific range is a range that does not include or barely includes portions where boundary positions are highly likely misaligned, and the boundaries within the non-specific range are less likely misaligned. The interpolation processing unit 18 corrects, by spatial interpolation, the boundary positions within the specific range (i.e., boundaries highly likely misaligned) based on the boundary positions within the non-specific range (i.e., boundaries less likely misaligned). Thus, the misalignment of boundary positions within the specific range can be corrected by the interpolation processing unit 18 before the user inputs correct positions of boundaries.

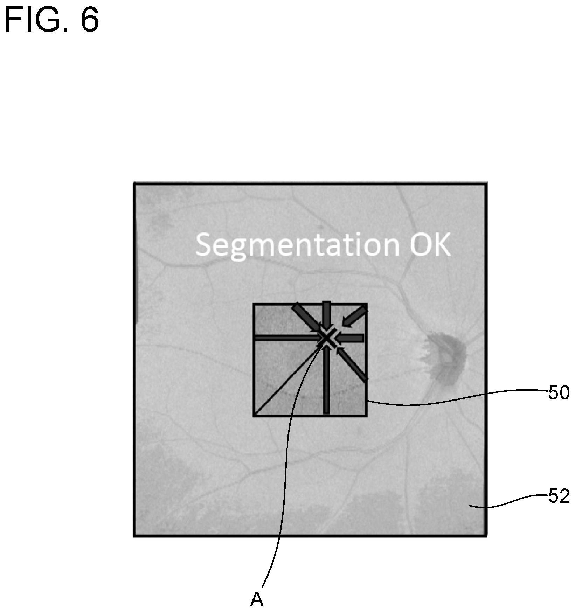

In the first spatial interpolation process of step S140, weighting may be varied in accordance with the distance from the non-specific range. FIG. 6 illustrates a specific range 50 and a non-specific range 52 in an en-face image of the subject eye. In FIG. 6, weighting of information of boundary within the non-specific range 52, which is used to spatially interpolate the position A on a boundary within the specific range 50, is depicted in different arrow thicknesses. The arrows are thicker as the distance between the position A and the non-specific range 52 decreases, while the arrows are thinner as the distance between the position A and the non-specific range 52 increases. The interpolation processing unit 18 increases the weight of information of boundary within the non-specific range 52 that are located closer to the position A, and decreases the weight of information of boundary within the non-specific range 52 that are located farther from the position A, to spatially interpolate and correct the position A on the boundary within the specific range 50.

After the first spatial interpolation process is completed, the processing unit 12 displays the three-dimensional image for which the first spatial interpolation process has been performed, on the display unit 32 (S150). The processing unit 12 may display, together with the three-dimensional image, an en-face image and a tomographic image on the display unit 32.

Subsequently, the interpolation processing unit 18 determines whether a correction completion instruction has been input or not (S160). The user checks boundary positions within the specific range in the three-dimensional image displayed on the display unit 32, and, if determining that the boundary positions are not misaligned, instructs a correction completion using the input unit 34. When the correction completion instruction is input (YES in step S160), the process of correcting layer boundaries in the tomographic images of the subject eye, which is illustrated in FIG. 5, is terminated. When the correction completion instruction is not input (NO in step S160), the processing unit 12 determines whether correct positions of boundaries have been input (S170). The user checks boundary positions within the specific range in the three-dimensional image displayed on the display unit 32, and, if determining that boundary positions are misaligned, inputs correct positions (i.e., corrected positions) of the boundaries using the input device 34. When the corrected positions of the boundaries are not input (NO in step S170), the flow returns to step S160, and steps S160 and S170 are repeated. Since steps S160 and S170 are the same as steps S22 and S24 in the first embodiment, detailed description thereof is omitted.

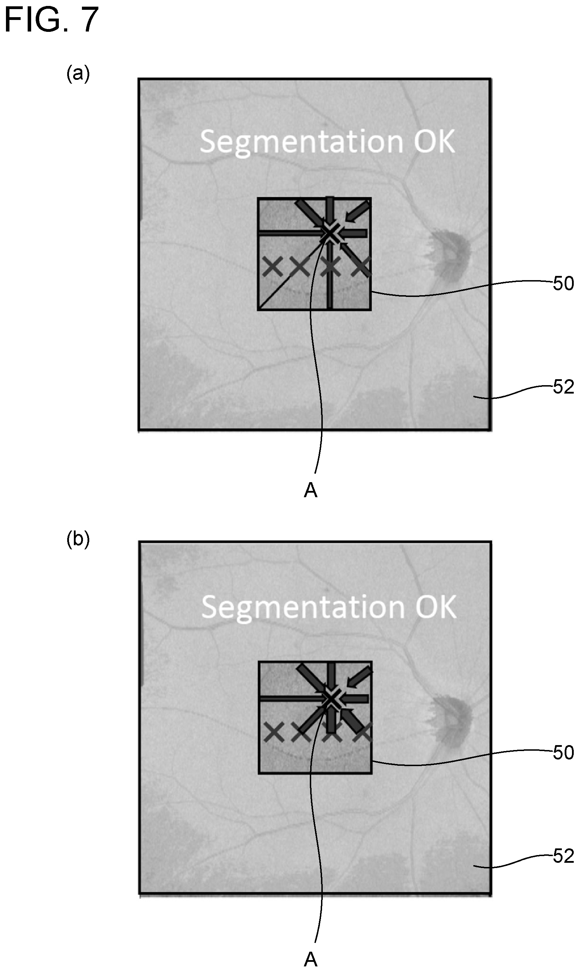

After the corrected positions of the boundary are input (YES in step S170), the interpolation processing unit 18 performs a spatial interpolation process (hereinafter referred to as a second spatial interpolation process) for boundary positions within the specific range based on the corrected positions of the boundary input in step S170 and the boundary positions within the non-specific range (S180). For example, it is assumed that the boundary positions within the specific range 50 have been corrected at the four locations indicated by "x" in FIG. 7(a). In this case, as illustrated in FIG. 7(b), the interpolation processing unit 18 uses information of the boundary positions within the non-specific range 52 when the position A within the specific range 50 is closer to the non-specific range 52 than to the corrected positions indicated by the mark "x", while the interpolation processing unit 18 uses information of the corrected positions indicated by the mark "x" to spatially interpolate and correct the boundary position at the position A when the position A is closer to the corrected positions indicated by the mark "x" than to the non-specific range. As illustrated in FIG. 7(b), in the second spatial interpolation process of step S180 as well, weighting may be varied in accordance with the distance from the corrected positions of boundary or the non-specific range.

After the spatial interpolation process is completed, the processing unit 12 displays the three-dimensional image for which the second spatial interpolation process has been performed, on the display unit 32 (S190). Subsequently, the flow returns to step S160, and steps S160 to S190 are repeated. That is, the user checks the three-dimensional image for which the second spatial interpolation process has been performed, and if boundary positions are still misaligned, inputs corrected positions for the boundaries again. Then, the processing unit 12 again spatially interpolates and corrects the boundary positions based on the corrected positions of the boundary. These steps are repeated until the user determines that there is no longer any misalignment of boundary positions (until the determination of YES is made in step S160).

In the present embodiment, the processing unit 12 corrects, by spatial interpolation, boundary positions within the specific range based on boundary positions within the non-specific range, before the user corrects boundary positions. Thus, the boundary positions within the range where boundary positions are highly likely misaligned are corrected, and misalignment of boundary positions determined in the segmentation process is reduced. Therefore, the number of locations where the user needs to correct boundary positions can be reduced, thereby reducing the user's workload.

### (Fifth Embodiment)

In the first to fourth embodiments above, only the normal tomographic images are used for correction of boundary positions; however, polarization tomographic images may be used. For example, the processing unit 12 may display a polarization tomographic image in addition to a normal tomographic image on the display unit 32 for the input of the corrected positions for boundary (e.g., in step S24 of FIG. 2 and step S170 of FIG. 5).

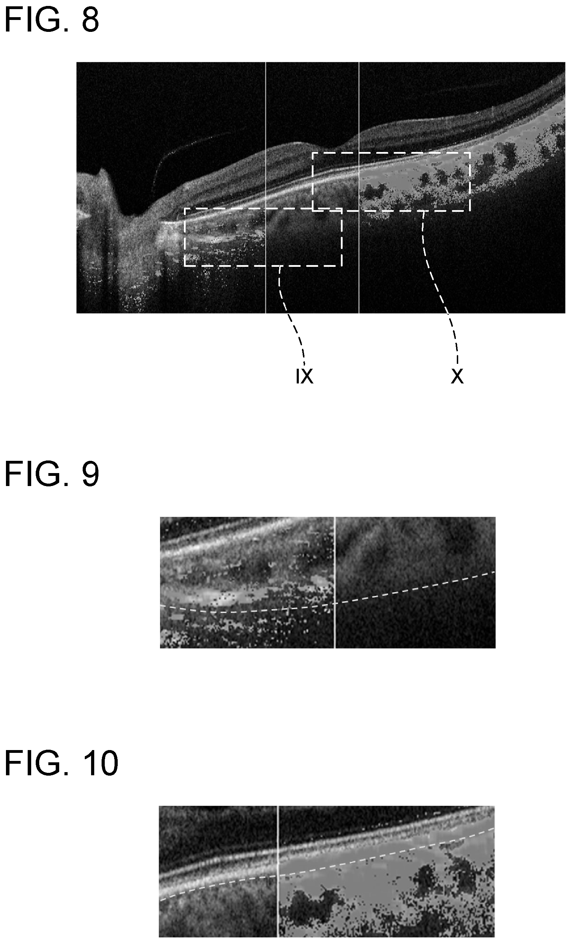

FIG. 8 illustrates an exemplary image in which a normal tomographic image at a cross section, a tomographic image indicating birefringence at the same cross section, and a tomographic image indicating entropy at the same cross section are concurrently displayed. In FIG. 8, a part of the image (specifically, the central portion) is displayed in the normal tomographic image at the cross section, another part thereof (specifically, its left portion) is displayed in the normal tomographic image with the tomographic image indicating birefringence at the same cross section superimposed thereon, and yet another part thereof (specifically, its right portion) is displayed in the normal tomographic image with the tomographic image indicating entropy at the same cross section superimposed thereon. FIG. 9 illustrates the normal tomographic image with the tomographic image indicating birefringence superimposed thereon (left) and the normal tomographic image (right). FIG. 10 illustrates the normal tomographic image (left) and the normal tomographic image with the tomographic image indicating entropy superimposed thereon (right). The broken lines in FIGS. 9 and 10 indicate layer boundaries.

As illustrated in FIG. 9, the user may have difficulty in determining the layer boundary in the normal tomographic image (right). In contrast, in the normal tomographic image with the tomographic image indicating birefringence superimposed thereon (left), birefringence intensity of in-layer tissue is different from birefringence intensity at the layer boundary, facilitating determination on the layer boundary. Further, as illustrated in FIG. 10, in the normal tomographic image with the tomographic image indicating entropy superimposed thereon (right) as well, an entropy value of in-layer tissue is different from an entropy value of the layer boundary, facilitating determination on the layer boundary. Thus, by using the normal tomographic image and the polarization tomographic images, the amount of information the user can refer to is increased, and the user can easily determine the layer boundary as compared with using only the normal tomographic image. Accordingly, the user can correct the boundary position more accurately.

How the normal tomographic image at the cross section, the tomographic image indicating birefringence at the same cross section, and the tomographic image indicating entropy at the same cross section are displayed is not particularly limited. For example, the normal tomographic image, the tomographic image indicating birefringence, and the tomographic image indicating entropy may be displayed separately in the same screen. Alternatively, the normal tomographic image at cross section, the tomographic image indicating birefringence at the same cross section, and the tomographic image indicating entropy at the same cross section may be displayed on the display unit 32 in a switchable manner.

In the present embodiment, a normal tomographic image and polarization tomographic images are used for input of corrected positions for boundary (e.g., in step S24 of FIG. 2 and step S170 of FIG. 5); however, the present disclosure is not limited thereto. For example, both a normal tomographic image and a polarization tomographic image may be used in the segmentation process (e.g., in step S14 of FIG. 2 and step S120 of FIG. 5).

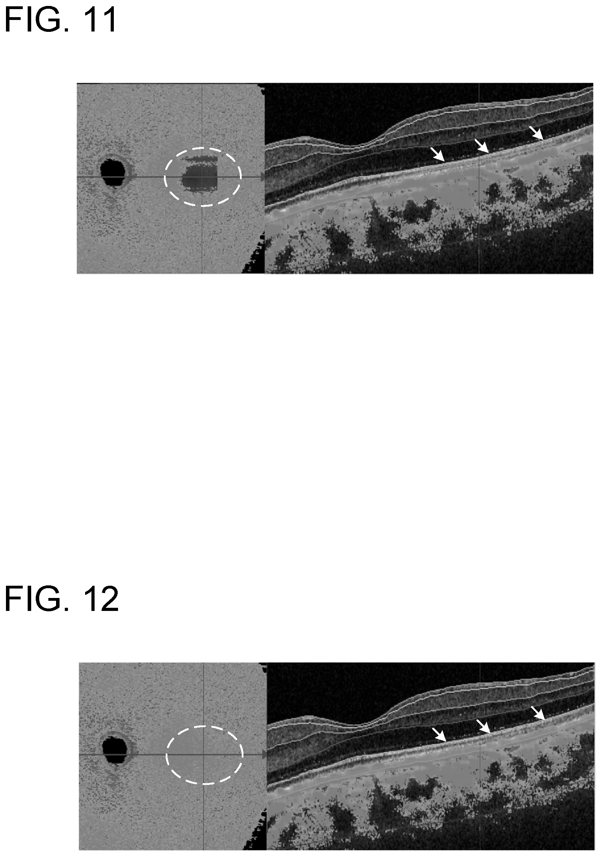

The processing unit 12 may use a normal tomographic image and a polarization tomographic image in specifying a specific range (e.g., in step S18 of FIG. 2 and step S130 of FIG. 5). FIG. 11 illustrates an en-face image at uncorrected boundary position (left) and a tomographic image at the cross section indicated by the arrows in the en-face image (right). FIG. 11 is the normal tomographic image with a tomographic image indicating entropy superimposed thereon, where a boundary specified in the segmentation process (step S14 of FIG. 2 and S120 of FIG. 5) is further superimposed. The boundary position is misaligned at the locations indicated by the arrows in the tomographic image (right). As indicated by the broken-line circle in the en-face image (left), when the boundary position is misaligned, the entropy value may be increased as compared with the surroundings. As described above, since the entropy value of in-layer tissue differs from the entropy value of a layer boundary, the entropy value differs between the locations where the boundary position is misaligned (in-layer tissue) and locations where the boundary position is correct (the layer boundary). The processing unit 12 evaluates a range having a significantly different entropy value (the range indicated by the broken-line circle in FIG. 11) in the en-face image at the boundary specified in the segmentation process as having low boundary positional accuracy, and specifies it as a specific range (in step S18 of FIG. 2 and step S130 of FIG. 5). The user corrects the boundary position within the specified specific range. When the user corrects the boundary position at the locations where the boundary position is misaligned in FIG. 11, the image shown in FIG. 12 is obtained. As illustrated in FIG. 12, after the boundary position is corrected, the entropy value is substantially the same as that of the surroundings, as indicated by the broken-line circle in the en-face image (right).

In this case as well, by using the normal tomographic image and the polarization tomographic image, information that can be referred to for boundary positions is increased. Accordingly, the processing unit 12 can more accurately specify a range where a boundary position is highly likely misaligned, as compared with using only a normal tomographic image.

In the examples above, the processing unit 12 specifies a specific range; however, the present disclosure is not limited thereto. For example, the processing unit 12 may correct boundary positions with low positional accuracy from a normal tomographic image and a polarization tomographic image (e.g., a tomographic image indicating entropy). For example, the processing unit 12 shifts the boundary positions with low accuracy to positions having substantially the same entropy as the surroundings. In this case, the processing unit 12 may inform the user of the automatically corrected boundary locations, and prompt the user to check if the corrected boundary positions are correct or not. The method of informing the user of the automatically corrected boundary locations is not particularly limited. For example, the processing unit 12 may surround the automatically corrected boundary locations by marks such as circles or squares, or may point them with arrows. Alternatively, the processing unit 12 may display the automatically corrected boundary locations in a different color from the color used for uncorrected locations.

In the present embodiment, boundary positions are corrected using not only the normal tomographic images but also polarization tomographic images. This allows for more accurate detection of boundary misalignment which is difficult to detect only based on the normal tomographic images. Thus, boundary misalignment can be corrected more accurately. Since boundary positions are corrected more accurately, uncorrected boundaries (i.e., uncorrected boundaries in the same tomographic image and boundaries in uncorrected tomographic images) can be also accurately corrected when the interpolation process performed.

In the present embodiment, the tomographic image indicating birefringence and the tomographic image indicating entropy are used as polarization tomographic images; however, polarization tomographic images of other types (e.g., tomographic image indicating fiber directions) may be used. In the present embodiment as well, the processing unit 12 may specify a specific tomographic image from among the plurality of tomographic images using the normal tomographic images and polarization tomographic images, instead of specifying a specific range. The processing unit 12 may perform the interpolation processes (e.g., step S26 of FIG. 2, and steps S140 and S180 of FIG. 5) using the normal tomographic images and polarization tomographic images.

Specific examples of the disclosure herein have been described in detail, however, these are mere exemplary indications and thus do not limit the scope of the claims. The art described in the claims includes modifications and variations of the specific examples presented above. Technical features described in the description and the drawings may technically be useful alone or in various combinations, and are not limited to the combinations as originally claimed. Further, the purpose of the examples illustrated by the present description or drawings is to satisfy multiple objectives simultaneously, and satisfying any one of those objectives gives technical utility to the present disclosure.

## Claims

1. An image processing apparatus comprising:
an acquisition unit configured to acquire a plurality of tomographic images obtained by imaging cross-sections at different positions of a subject eye;
a display unit configured to display a tomographic image acquired by the acquisition unit;
a specifying unit configured to be operated by a user and to specify boundary positions between layers in the tomographic image displayed on the display unit; and
a processing unit configured to perform an interpolation process to interpolate and determine, when an unspecified tomographic image in which boundary positions have not been specified is present between two specified tomographic images of cross-sections adjacent thereto among specified tomographic images in which the boundary positions have been specified by the specifying unit, a boundary of the unspecified tomographic image based on boundaries in the two specified tomographic images.

2. The image processing apparatus according to claim 1,
wherein the processing unit is further configured to perform a segmentation process to determine a boundary between layers by performing segmentation using machine learning on each of the plurality of tomographic images acquired by the acquisition unit,
the display unit displays a tomographic image on which the boundary determined in the segmentation process is superimposed, and
the specifying unit instructs correction of the boundary positions superimposed on the tomographic image displayed on the display unit.

3. The image processing apparatus according to claim 2,
wherein the plurality of tomographic images comprises a non-polarization tomographic image indicating tissues in the subject eye by scattering intensity and a polarization tomographic image indicating a polarization state in the subject eye, and
the processing unit performs the segmentation process based on the non-polarization tomographic image and the polarization tomographic image.

4. The image processing apparatus according to claim 2,
wherein the processing unit is further configured to perform, on each of the tomographic images in which the boundary has been determined in the segmentation process, a specific-range specifying process to specify a specific range for correction of the boundary positions.

5. The image processing apparatus according to claim 4,
wherein the processing unit is further configured to calculate reliability information related to reliability of the boundary positions determined in the segmentation process, and
in the specific-range specifying process, the processing unit specifies the specific range based on the calculated reliability information such that low-reliability boundary positions are included therein.

6. The image processing apparatus according to claim 4,
wherein when positions of two adjacent pixels on a boundary determined in the segmentation process differ in a depth direction by a predetermined value or more, the processing unit specifies the specific range in the specific-range specifying process such that the two adjacent pixels are included therein.

7. The image processing apparatus according to claim 4,
wherein in the specific-range specifying process, the processing unit specifies the specific range such that pixels having a luminance value equal to or less than a predetermined value on a boundary determined in the segmentation process are included therein.

8. The image processing apparatus according to claim 4,
wherein the processing unit is further configured to perform a poor-fixation detection process to detect a location of poor fixation in each of the plurality of tomographic images, and
the processing unit specifies the specific range such that the location of poor fixation detected in the poor-fixation detection process is included therein.

9. The image processing apparatus according to claim 4,
wherein the plurality of tomographic images comprises a non-polarization tomographic image indicating tissues in the subject eye by scattering intensity and a polarization tomographic image indicating a polarization state in the subject eye, and
the processing unit performs the specific-range specifying process based on the non-polarization tomographic image and the polarization tomographic image.

10. The image processing apparatus according to claim 2,
wherein the processing unit is configured to perform a specific tomographic image specifying process to specify a specific tomographic image in which boundary positions are to be corrected from among the plurality of tomographic images.

11. The image processing apparatus according to claim 10,
wherein the processing unit is further configured to calculate reliability information related to reliability of the boundary positions determined in the segmentation process, and
in the specific tomographic image specifying process, the processing unit specifies, based on the calculated reliability information, a tomographic image including low-reliability boundary positions as the specific tomographic image.

12. The image processing apparatus according to claim 10,
wherein in the specific tomographic image specifying process, the processing unit specifies a tomographic image in which positions of two adjacent pixels on a boundary determined in the segmentation process differ in a depth direction by a predetermined value or more as the specific tomographic image.

13. The image processing apparatus according to claim 10,
wherein in the specific tomographic image specifying process, the processing unit specifies a tomographic image that includes pixels having a luminance value equal to or less than a predetermined value on a boundary determined in the segmentation process as the specific tomographic image.

14. The image processing apparatus according to claim 10,
wherein the processing unit is further configured to perform a poor-fixation detection process to detect a location of poor fixation in each of the plurality of tomographic images, and
the processing unit specifies a tomographic image including the poor fixation detected in the poor-fixation detection process as the specific tomographic image.

15. The image processing apparatus according to claim 10,
wherein the plurality of tomographic images comprises a non-polarization tomographic image indicating tissues in the subject eye by scattering intensity and a polarization tomographic image indicating a polarization state in the subject eye, and
the processing unit performs the specific tomographic image specifying process based on the non-polarization tomographic image and the polarization tomographic image.

16. The image processing apparatus according to claim 1,
wherein the specifying unit specifies, in accordance with user operation, a specific range within the subject eye in the tomographic image and instructs correction of boundary positions within the specified specific range.

17. The image processing apparatus according to claim 1,
wherein the plurality of tomographic images comprises a non-polarization tomographic image indicating tissues in the subject eye by scattering intensity and a polarization tomographic image indicating a polarization state in the subject eye, and
the display unit displays the non-polarization tomographic image and the polarization tomographic image.

18. An image processing apparatus comprising:
an acquisition unit configured to acquire a three-dimensional image generated from a plurality of tomographic images obtained by imaging cross-sections at different positions of a subject eye;
a display unit configured to display the three-dimensional image acquired by the acquisition unit;
a specifying unit configured to be operated by a user and to specify boundary positions between layers in the three-dimensional image displayed on the display unit; and
a processing unit,
wherein the processing unit is configured to perform on the three-dimensional image acquired by the acquisition unit:
a specific-range specifying process to specify a specific range for correction of boundary positions; and
a spatial interpolation process to spatially interpolate and correct the boundary in the specific range based on boundary positions in a non-specific range which is not specified in the specific-range specifying process,
the specifying unit specifies the boundary in the three-dimensional image after the spatial interpolation process has been performed, and
the processing unit is further configured to spatially interpolate and determine the boundary in the specific range based on the boundary specified by the specifying unit.

19. An image processing apparatus comprising:
an acquisition unit configured to acquire a plurality of tomographic images obtained by imaging cross-sections at different positions of a subject eye;
a processing unit configured to:
perform, on each of the plurality of tomographic images acquired by the acquisition unit, a segmentation process to determine a boundary between layers by performing segmentation using machine learning; and
perform, on each of tomographic images in which the boundary has been determined, a specific-range specifying process to specify a specific range for correction of the boundary positions;
a display unit configured to display a tomographic image acquired by the acquisition unit and the specific range specified by the processing unit; and
a correction instruction unit configured to be operated by a user and to instruct correction of the boundary positions within the specific range in the tomographic image displayed on the display unit.

20. An optical coherence tomographic apparatus comprising:
an imaging capturing unit configured to capture tomographic images along cross-sections at different positions of a subject eye; and
the image processing apparatus according to any one of claims 1 to 19, configured to process the tomographic images captured by the imaging capturing unit.

21. A computer program for processing a plurality of tomographic images obtained by imaging cross-sections at different positions of a subject eye,
wherein the computer program causes a computer to function as:
an acquisition unit configured to acquire the plurality of tomographic images;
a receiving unit configured to receive from a user a specifying instruction to specify boundary positions between layers in each of the tomographic images; and
an interpolation unit configured to interpolate and determine, when an unspecified tomographic image in which boundary positions have not been specified is present between two specified tomographic images of cross-sections adjacent thereto among specified tomographic images in which the boundary positions have been specified according to the specifying instruction received from the receiving unit, a boundary of the unspecified tomographic image based on boundaries in the two specified tomographic images.
